**Europäisches Patentamt**

**European Patent Office**

**Office européen des brevets**

(19)

(11) Veröffentlichungsnummer: **0 100 419**

**B1**

(12) # EUROPÄISCHE PATENTSCHRIFT

(45) Veröffentlichungstag der Patentschrift:
02.09.87

(21) Anmeldenummer: **83105979.5**

(22) Anmeldetag: **18.06.83**

(51) Int. Cl.⁴: **A 01 N 1/02, A 61 K 35/18**

(54) **Wässrige Lösung zum Suspendieren und Lagern von Zellen, insbesondere Erythrozyten.**

(30) Priorität: **07.07.82 DE 3225408**

(43) Veröffentlichungstag der Anmeldung:
**15.02.84 Patentblatt 84/7**

(45) Bekanntmachung des Hinweises auf die Patenterteilung:
**02.09.87 Patentblatt 87/36**

(84) Benannte Vertragsstaaten:
**BE DE FR GB NL**

(56) Entgegenhaltungen:
**DD-A-152 719**
**GB-A-970 190**
**US-A-4 267 269**

**Die Akte enthält technische Angaben, die nach dem Eingang der Anmeldung eingereicht wurden und die nicht in dieser Patentschrift enthalten sind.**

(73) Patentinhaber: **Biotest- Serum- Institut GmbH, Flughafenstrasse 4, D-6000 Frankfurt- Niederrad (DE)**

(72) Erfinder: **Walker, Wolfram H., Dr. Dipl.- Chem., Leipziger Strasse 12, D-6074 Rödermark (DE)**
Erfinder: **Gänshirt, Karlheinz, Dr. Dipl.- Chem., August- Bebel- Strasse 34, D-6072 Dreieich (DE)**

(74) Vertreter: **Beil, Hans Christoph, Dr., Beil, Wolff und Beil Rechtsanwälte Adelonstrasse 58, D-6230 Frankfurt am Main 80 (DE)**

EP 0 100 419 B1

## Beschreibung

Die Erfindung betrifft eine wäßrige Lösung zum Suspendieren und Lagern von Erythrozyten, enthaltend Natriumchlorid, Glucose oder Fructose, Adenin und einen Zuckeralkohol.

Im Rahmen der Blutkomponententherapie wird aus stabilisiertem Vollblut (Blutkonserve) durch Abtrennen des Plasmas ein Erythrozytenkonzentrat hergestellt. Dieses Erythrozytenkonzentrat wird heute in einer großen Zahl von therapeutischen Anwendungen eingesetzt, bei denen die Indikation Anämie bzw. Erythrozytenzufuhr besteht. Da durch die Abtrennung des Plasmas auch ein Teil der einer Blutkonserve üblicherweise zugesetzten Blutstabilisatorlösung entfernt wird, ist man dazu übergegangen, die Konzentrationen bestimmter Bestandteile dieser Stabilisatorlösungen zu erhöhen, um dieselbe Lagerfähigkeit des Erythrozytenkonzentrates wie die für die Blutkonserve zu erhalten. Es ist außerdem bekannt, daß durch eine nachträgliche Zugabe einer Lösung zum Suspendieren zu dem Erythrozytenkonzentrat die ursprünglich hergestellten Konzentrate durch diese Suspensionslösung verdünnt werden, so daß eine erwünschte niedrige Viskosität erreicht und dadurch die Transfusion dieser Lösung erleichtert wird. Sie ermöglicht außerdem, spezifische für die Lagerungsfähigkeit der Erythrozyten notwendige Substanzen zuzuführen, um deren Überlebensrate in vitro und in vivo zu verlängern.

Als Lösung zum Suspendieren und Lagern von Erythrozyten, eignen sich verschiedene Lösungen. So wird z. B. in der US-PS- 4 267 269 eine Lösung beschrieben, die neben Natriumchlorid, Glucose oder Fructose und Adenin, den Zuckeralkohol Mannit enthält.

Aus der DD-A-152 719 ist ein Verfahren zur Konservierung von Erythrozyten bekannt, bei dem man den Erythrozyten eine Konservierungslösung zusetzt, die Glucose, Saccharose, Zitrat und NaCl und ggf. Adenin, Guanosin und Zuckeralkohole, vorzugsweise Mannit, enthält.

Aus der GB-A-970 190 sind Konservierungslösungen für lebende Tierzellen enthaltendes Material, wozu auch Blut gehört, bekannt, die eine Mischung aus verschiedenen Zuckeralkoholen enthalten. Diese Lösungen dienen jedoch offensichtlich nicht zur Konservierung von Erythrozytenkonzentraten, sondern zur Konservierung von Vollblut.

Zwar wurde durch Zugabe solcher Suspensionslösungen zu Erythrozytenkonzentraten die Lagerungsfähigkeit und damit die Überlebensrate der Erythrozyten verbessert, jedoch waren diese Verbesserung und insbesondere die Hämolyserate noch nicht voll zufriedenstellend.

Der Erfindung lag die Aufgabe zugrunde, die Lagerungsfähigkeit und damit die Überlebensrate bzw. die Hämolyserate bei Erythrozyten in einem Erythrozytenkonzentrat zu verbessern.

Diese Aufgabe wird erfindungsgemäß dadurch gelöst, daß man eine Lösung verwendet, die als Zuckeralkohol Sorbit oder Xylit und außerdem Guanosin enthält.

Überraschenderweise wurde gefunden, daß eine Lösung zum Suspendieren und Lagern von Erythrozyten, in günstigerer Weise auf die Überlebensrate und ganz besonders auf die Hämolyserate von Erythrozyten einwirkt, wenn sie anstelle von Mannit den Zuckeralkohol Sorbit oder Xylit enthält. Die Hämolyserate ist ein wichtiger Parameter zur Bestimmung der Qualität der Erythrozyten. Unter Hämolyserate versteht man die Geschwindigkeit der Hämolyse. Wünschenswert ist somit eine möglichst geringe Hämolyserate.

Diese günstigen Eigenschaften dieser Lösung lassen sich durch Zugabe von Guanosin alleine oder in Kombination mit einem oder mehreren Phosphaten noch weiter verbessern. Besonders geeignet ist eine Kombination aus einem Hydrogenphosphat und einem Dihydrogenphosphat, die als Puffer wirkt.

Eine weitere Verbesserung der Lagerungsfähigkeit der Erythrozyten läßt sich dadurch erreichen, daß man den vorstehend genannten Mischungen ein Kolloid, beispielsweise modifizierte Gelatine, Dextran, Hydroxyethylstärke oder Serumalbumin zusetzt (vgl. Ullmann, 4. Aufl., Bd. 8 (1974), S. 634-640).

Typische Lösungen setzen sich beispielsweise wie folgt zusammen:

Pro 1000 ml gelöst in aqua ad injectabilia 0,5 bis 20 g Natriumchlorid, 0 bis 5 g Di-natriumhydrogenphosphat, 0 bis 5 g Natriumdihydrogenphosphat, 0,5 bis 20 g Glucose oder Fructose, 1 bis 20 g Sorbit oder Xylit, 0,05 bis 1 g Adenin, bis 1,5 g Guanosin und 0 bis 10 % Kolloide, vorzugsweise 2,5 bis 6,5 g Natriumchlorid, 0,7 bis 2,5 g Di-natriumhydrogenphosphat x $2H_2O$, 0,7 bis 2,5 g Natriumdihydrogenphosphat x $H_2O$, 5 bis 15 g Glucose oder Fructose, vorzugsweise Glucose-Monohydrat, 5 bis 15 g Sorbit oder Xylit, vorzugsweise Sorbit, 0,1 bis 0,5 g Adenin-Hydrochlorid und 0,1 bis 0,5 g Guanosin;

besonders bevorzugt 4,2 g Natriumchlorid, 1,14 g Di-natriumhydrogenphosphat x $2H_2O$, 1,11 g Natriumdihydrogenphosphat x $H_2O$, 9,4 g Glucose-Monohydrat, 10,0 g Sorbit, 0,25 g Adenin-Hydrochlorid und 0,4 g Guanosin.

Bei der Anwendung dieser Lösungen wird üblicherweise so verfahren, daß zunächst ein mit einer ACD-, CPD- oder Heparin-Lösung (USP XX (1980), S. 49 - 50) stabilisiertes Vollblut hergestellt wird. Die Vollblutkonserve wird zentrifugiert und anschließend das Plasma abgetrennt. Anschließend wird vorzugsweise im geschlossenen, aus mehreren Beuteln bestehenden System zum Erythrozytenkonzentrat Suspensionslösung zum Waschen oder zum Lagern zugegeben. Zum Beispiel können zu ca. 200-300 ml Erythrozytenkonzentrat mit HK von 60-85, ca. 50 bis 250 ml Suspensionslösung in einer

oder mehreren Portionen zugegeben werden. Die Mischung kann dann wie bisher eine Blutkonserve im Kühlschrank bei ca. 6° C gelagert oder auch direkt transfundiert werden.

Die erfindungsgemäßen Lösungen können auch zur Waschung und Konservierung von Erythrozyten verwendet werden. Gleichzeitig sind diese Mischungen zur Transfusion geeignet. Bei der Anwendung dieser Lösungen als Waschlösung werden z. B. zu 250 ml Erythrozytenkonzentrat 50 bis 250 ml dieser Lösungen, vorzugsweise 70 ml, hinzugegeben. Die Mischungen werden zentrifugiert und der Überstand abgetrennt. Dieser Vorgang kann 2 bis 3 mal wiederholt werden, so daß beim Waschprozeß bis zu 210 ml dieser Lösungen notwendig sind. Anschließend kann das Erythrozytenkonzentrat zusammen mit der Suspensionslösung gelagert oder aber mit oder ohne Suspensionslösung sofort transfundiert werden.

### Beispiel 1

#### Herstellung einer Suspensionslösung

4,2 g Natriumchlorid, 1,14 g Di-natriumhydrogenphosphat x $2H_2O$, 1,11 g Natriumdihydrogenphosphat x $H_2O$, 9,4 g Glucose-Monohydrat, 10,0 g Sorbit, 0,25 g Adenin-Hydrochlorid und 0,4 g Guanosin werden in getrennten Behältern eingewogen. Etwa die Hälfte von 1000 ml aqua ad injectabilia (nach Ph.Eur.) wurde im vorsterilisierten doppelwandigen Ansatzkessel vorgelegt und auf ca. 40° C erwärmt. Unter Umrühren wurden die eingewogenen Bestandteile innerhalb von 5 Minuten zugegeben und unter gleichzeitiger Zugabe der restlichen Menge an aqua ad injectabilia etwa 15 Minuten lang gerührt. Die Temperatur der Lösung wurde hierbei auf ca. 40° C gehalten. Die Lösung wurde unter Druck durch Membranfilter mit 0,2 μm Porengröße in die Behälter sterilfiltriert, aus denen mittels Pumpen eine Abfüllung der einzelnen Kunststoffbeutel erfolgte.

### Beispiel 2

In einem Dreifach-Blutbeutelsystem wurden 500 ml Blut mit 70 ml CPD-Blutstabilisatorlösung stabilisiert. Die Konserve wurde auf ca. 6° C gekühlt und nach 24 Stunden zentrifugiert. Anschließend wurden ca. 300 ml Plasma in einen Leerbeutel übergedrückt. Zum Erythrozytenkonzentrat wurden ca. 90 ml der nach Beispiel 1 hergestellten Suspensionslösung aus dem Satellitenbeutel hinzugegeben, so daß die Endmischung ca. 370 ml betrug.

Es wurde festgestellt, daß diese Mischung besonders günstige Hämolyseraten zeigt. Sie waren vergleichbar mit denen der ursprünglichen Blutkonserve, die unter denselben Bedingungen lagerte. Typische Lagerzeiten für diese Suspensionslösungen betrugen 3 bis 6 Wochen bei ca. 6° C.

### Patentansprüche

1. Wäßrige Lösung zum Suspendieren und Lagern von Erythrozyten enthaltend Natriumchlorid, Glucose oder Fructose, Adenin und einen Zuckeralkohol, dadurch gekennzeichnet, daß sie als Zuckeralkohol Sorbit oder Xylit und außerdem, Guanosin enthält.

2. Lösung nach Anspruch 1, dadurch gekennzeichnet, daß sie außerdem ein oder mehrere Phosphate enthält.

3. Lösung nach einem der Ansprüche 1 oder 2, dadurch gekennzeichnet, daß sie pro 1 aqua ad injectabilia 0,5 bis 20 g Natriumchlorid, 0 bis 5 g Dinatriumhydrogenphosphat, 0 bis 5 g Natriumdihydrogenphosphat, 0,5 bis 20 g Glucose oder Fructose, 1 bis 20 g Sorbit oder Xylit, 0,05 bis 1 g Adenin und bis 1,5 g Guanosin enthält.

4. Lösung nach Anspruch 3, dadurch gekennzeichnet, daß sie pro 1 aqua ad injectabilia 4,2 g Natriumchlorid, 1,14 g Di-natriumhydrogenphosphat x $2H_2O$, 1,11 g Natriumdihydrogenphosphat x $H_2O$, 9,4 g Glucose-Monohydrat, 10,0 g Sorbit, 0,25 g Adenin-Hydrochlorid und 0,4 g Guanosin enthält.

5. Lösung nach einem der vorstehenden Ansprüche, dadurch gekennzeichnet, daß sie außerdem ein Kolloid enthält.

### Claims

1 Aqueous solution for suspending and storring erythrocytes, containing sodium chloride, glucose or fructose, adenine, and a sugar alcohol, as sugar alcohol and additionally contains sorbitol or xylitol as sugar alacohol and additionally contains guanosine.

2. Solution according to Claim 1, characterized in that it additionally contains one or more phosphates.

3. Solution according to one of the Claims 1 or 2, characterized in that it contains 0.5-20 g of sodium chloride, 0-5 g of disodium hydrogenphosphate, 0-5 g of sodium dihydrogenphosphate, 0.5-20 g of glucose or fructose, 1-20 g of sorbitol or zylitol, 0.05,1 g of adenine, and up to 1.5 g of quanosine per liter of water for injections.

4. Solution according to Claim 3, characterized in that it contains 4.2 g of sodium chloride, 1.14 g of disodium hydrogenphosphate x $2H_2O$, 1.11 g sodium dihydrogenphosphate x $H_2O$, 9.4 g of glucose-monohydrate, 10.0 g of sorbitol, 0.25 g of adenine hydrochloride, and 0.4 g of guanosine per liter of water for injections.

5. Solution according to one of the precedent Claims characterized in that it additionally contains a colloid.

**Revendications**

1. Solution aqueuse pour la mise en suspension et la conservation d'érythrocytes, contenant du chlorure de sodium, du glucose ou du fructose, de l'adénine et un alcool de sucre, caractérisée en ce qu'elle contient comme alcool de sucre du sorbitol ou du xylitol et en outre de la guanosine.

2. Solution selon la revendication 1, caractérisée en ce qu'elle contient en outre un ou plusieurs phosphates.

3. Solution selon l'une des revendications 1 ou 2, caractérisée en ce qu'elle contient pour un litre d'eau injectable de 0,5 à 20 g de chlorure de sodium, de 0 à 5 g de phosphate acide di-sodique, de 0 à 5 g de phosphate diacide de sodium, de 0,5 à 20 g de glucose ou de fructose, de 1 à 20 g de sorbitol ou de xylitol, de 0,05 à 1 g d'adénine et jusqu'à 1,5 g de guanosine.

4. Solution selon la revendication 3, caractérisée en ce qu'elle contient pour un litre d'eau injectable, 4,2 g de chlorure de sodium, 1,14 g de phosphate acide di-sodique · $2H_2O$, 1,11 g de phosphate diacide de sodium · $H_2O$, 9,4 g de glucose mono-hydraté, 10,0 g de sorbitol, 0,25 g de chlorhydrate d'adénine et 0,4 g de guanosine.

5. Solution selon l'une des revendications précédentes, caractérisée en ce qu'elle contient en outre un colloïde.